# EUROPEAN PATENT APPLICATION

(11) **EP 4 252 762 A1**
(43) Date of publication of application: **04.10.2023**
(21) Application number: 21898670.1
(22) Date of filing: 26.11.2021
(51) Int. Cl.: A61K 35/32, A61K 9/00, A61P 19/02, A61L 27/36, A61L 27/24

(54) **METHOD FOR PREPARING INJECTABLE INJECTION COMPOSITION DERIVED FROM ANIMAL CARTILAGE, AND USE THEREOF**

(30) Priority: 27.11.2020 KR 20200163201
(60) Divisional application: 23183127.2
(71) Applicant: Kangstem Biotech Co., Ltd., Seoul 06179 (KR)
(72) Inventor: KANG, Kyung Sun, Seoul 06338 (KR); LEE, Seunghee, Seoul 08797 (KR); AHN, Jong Chan, Bucheon-si, Gyeonggi-do 14598 (KR); KIM, Mijin, Suwon-si, Gyeonggi-do 16387 (KR)
(74) Representative: Zwicker, Jörk
(86) International application number: PCT/KR2021/017617
(87) International publication number: WO 2022/114845

(57) **Abstract**

The present invention relates to a method of manufacturing an animal cartilage-derived injectable composition, an injectable composition manufactured by the method, and a use thereof.

The injectable composition of the present invention includes a collagen-containing biomaterial in a formulation injectable into joint cavity and may induce cartilage tissue regeneration by repairing tissue via direct injection to a target region without surgical incision. In addition, the injectable composition may be used as a therapeutic agent for arthritis by alleviating osteoarthritis not only because the animal cartilage-derived extracellular matrix contained in the injectable composition protects articular cartilage tissue but also because an environment capable of regenerating damaged articular tissue is created by inducing differentiation of intra-articular stem cells into chondrocytes.

## Description

### [Technical Field]

The present invention relates to a method of manufacturing an animal cartilage-derived injectable composition, an injectable composition manufactured by the method, and a use thereof.

### [Background Art]

As methods for treating damaged cartilage, debridement, bone marrow stimulating technique, osteochondral autograft, and the like have conventionally been used. Such methods are invasive methods generally used after damage to cartilage has significantly progressed, and a method of injecting hyaluronic acid products into a joint cavity is applicable in the early stages of cartilage damage. Most of the invasive treatment methods cause problems such as incision for surgery, collection of periosteum, complexity of use, expensive costs for treatment, leakage of adult stem cells induced during a process of stimulating bone marrow, and formation of abnormal fibrotic cartilage caused by hemostasis. In addition, the method of injecting a hyaluronic acid product only plays a simple role in alleviating pain by lubrication (Frizziero L, et al., Clinical and Experimental Rheumatology, 01 Jul 1998, 16(4):441-449).

Therefore, there is an urgent need to develop therapeutic agents having therapeutic efficacy beyond simple lubrication and enabling minimum invasive procedures to solve these problems.

Meanwhile, extracellular matrix (ECM) is a noncellular portion present in tissues as an aggregate of biopolymers that creates an environment capable of maintaining the original structure of living tissues by filling gaps between cells and physically supporting tissues. In particular, collagen, as a main component of the extracellular matrix of cartilage tissue, has been known to be a main component constituting tissue in cartilage and creating a microenvironment for cellular growth and differentiation.

### [Disclosure]

### [Technical Problem]

As a result of intensive efforts to treat damaged cartilage, the present inventors have found an animal cartilage-derived injectable composition capable of repairing tissue and regenerating cartilage tissue by directly injecting a biomaterial in an injectable for containing animal cartilage-derived collagen and extracellular matrix into a target region without surgical incision, thereby completing the present invention.

### [Technical Solution]

An object of the present invention is to provide a method of manufacturing an injectable composition for preventing or treating osteoarthritis.

Another object of the present invention is to provide an injectable composition for preventing or treating osteoarthritis manufactured by the method.

Still another object of the present invention is to provide a method of preventing or treating osteoarthritis including administering the injectable composition manufactured by the method to joint cavity of an individual.

### [Advantageous Effects]

The injectable composition of the present invention is a collagen-containing biomaterial in a formulation injectable into joint cavity and may induce cartilage tissue regeneration by repairing tissue via direct injection to a target region using a syringe without surgical incision. In addition, the injectable composition may be used as a therapeutic agent for arthritis by alleviating osteoarthritis not only because the animal cartilage-derived extracellular matrix contained in the injectable composition protects articular cartilage tissue but also because an environment capable of regenerating damaged articular tissue is created by inducing differentiation of intra-articular stem cells into chondrocytes.

### [Brief Description of Drawings]

FIG. 1 shows injectable raw materials and solubilization conditions.
FIG. 2 shows photographs of CAM solutions prepared under three solubilization temperature conditions (4°C, 25°C, and 37°C) for 6 hours.
FIG. 3 shows injectable sterilization conditions.
FIG. 4 is a schematic diagram of a method of manufacturing a porcine cartilage-derived injection (CAM solution) of the present invention.
FIG. 5 shows contents of proteins contained in a porcine cartilage-derived injection before and after sterilization identified via SDS-page.
FIG. 6 shows contents of collagen according to conditions of a solubilization process.
FIG. 7 shows the ability to differentiation into chondrocytes according to conditions of a solubilization process.
FIG. 8 shows the ability to regenerate cartilage tissue in an osteoarthritis model of rats induced by causing damage to the anterior cruciate ligament when umbilical cord blood mesenchymal stem cells are administered for 8 weeks (stem cell-administered group) or when the stem cells and a CAM solution prepared by the process shown in FIG. 5 are administered for 8 weeks (stem cell+CAM solution-administered group). (Scale bar = 100 µm).

### [Best Mode]

The present invention will be described in detail. Meanwhile, each description and embodiment disclosed in the present invention may be applied herein to describe different descriptions and embodiments. In other words, all combinations of various components disclosed in the present invention are included within the scope of the present invention. Furthermore, the scope of the present invention should not be limited by the detailed description provided below.

An aspect of the present invention to achieve the above-described objects provides a method of manufacturing an injectable composition for preventing or treating osteoarthritis.

Specifically, the method includes:
(a) solubilizing a composition including an animal cartilage-derived acellular extracellular matrix (cartilage acellular matrix, CAM) by stirring the composition at a temperature higher than 30°C and lower than 55°C; and
(b) preparing an injectable composition by centrifuging the aqueous solution prepared in step (a) and collecting a solution, without being limited thereto.

In the present invention, the step (a) may be a step of solubilizing the CAM-containing composition by stirring the composition at a temperature higher than 30°C and lower than 55°C.

As used herein, the term "cartilage acellular matrix (CAM)" may be used as a raw material of the injectable composition of the present invention. This may be manufactured by separation of cartilage from an animal and decellularization by an enzymatic or physiochemical method according to a process well known in the art or commercially purchased. For example, a product of ATEMs may be used. However, the embodiment is not limited thereto.

The CAM may be in various forms such as powder and sponge, without being limited thereto.

In addition, the animal from which the CAM is obtained is not particularly limited as long as the animal has cartilage tissue, but specifically the animal may be a pig.

In the present invention, the CAM may be sterilized.

Sterilization may be performed by any method well known in the art, for example, dry heat sterilization, moist heat sterilization, ethylene oxide (EO) gas sterilization, plasma sterilization, sterilization by filtration, or radiation sterilization using X-rays, E-beams, or gamma-rays. Specifically, in the present invention, sterilization may be performed by electron beam (E-beam) or gamma irradiation. More specifically, in the case where sterilization is performed using E-beams, E-beam irradiation at a dose of 15 to 25 kGy may be used and about 15 to 25 kGy of the E-beam may be E-beam of the VDmax15 method. Also, in the case where sterilization is performed using gamma-rays, gamma-rays at a dose of about 25 to 40 kGy may be used and about 25 to 40 kGy of the gamma-rays may be gamma-rays of the VDmax25 method. However, the embodiment is not limited thereto.

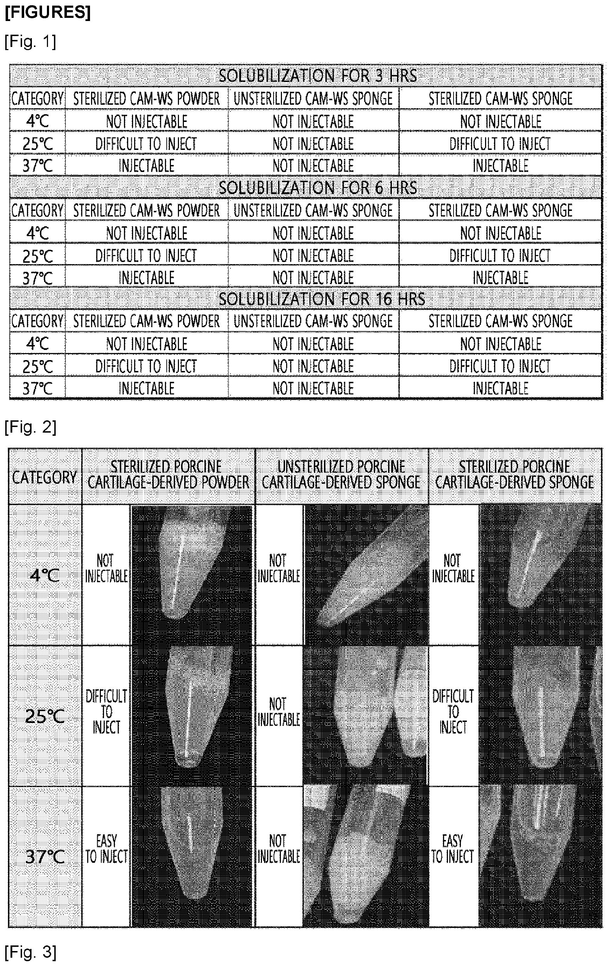

In an embodiment of the present invention, it was confirmed that the CAM sterilized by gamma-ray irradiation at a dose of 25 to 40 kGy of VDmax25 was prepared in an injectable formulation in the case of solubilizing the CAM under particular temperature and time conditions, but the unsterilized CAM was not injectable because it was not solubilized (FIGS. 1 and 2).

As used herein, the term "about" may be used in front of a particular numerical value. The term "about" used in the present invention is inclusive of not only the stated value following the term but also an acceptable range of deviation for the stated value. In consideration of the context in which the numerical value is provided, the acceptable range of deviation for the value may be determined. For example, the term "about" may refer to a range of -10% to +10% of a given numerical value. As another example, the term "about" may refer to a range of -5% to +5% of the numerical value. However, the embodiment is not limited thereto.

In the present invention, the term "solubilization" refers to a process of converting a non-injectable formulation into a water-soluble form to prepare an injectable formulation. The solubilization may be performed by stirring under particular temperature and time conditions.

Any method known in the art may be used for the stirring without limitation, for example, a shaking incubator may be used, but the embodiment is not limited thereto.

In the present invention, temperature conditions of step (a) may be a temperature higher than about 30°C and lower than 55°C, a temperature of about 33°C to 41°C, specifically, a temperature of about 37°C.

Under temperature conditions lower than the above-described temperature conditions, a viscosity of the prepared solution increases failing to produce an injectable formulation. Under temperature conditions higher than the above-described temperature conditions, the content of collagen significantly decreases in the produced solution and differentiation of stem cells into chondrocytes is not induced, resulting in a decrease in preventive or therapeutic effects on osteoarthritis.

In the present invention, the solubilization of step (a) may be performed for less than about 24 hours, for less than about 20 hours, specifically, for about 6 to 18 hours.

In the case of performing the solubilization for a shorter time than the above-described time conditions, the composition may not be solubilized and the viscosity of the produced solution increases failing to produce an injectable formulation. In the case of performing the solubilization for a longer time than the above-described time conditions, the content of collagen, as an active ingredient, significantly decreases in the injectable composition so that the effect of the injectable composition on alleviating osteoarthritis may be reduced.

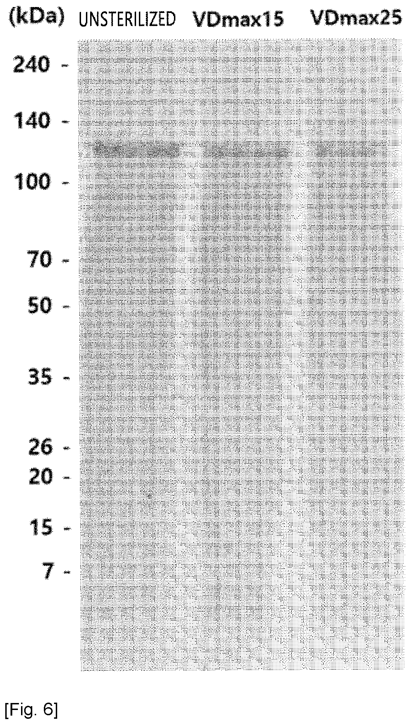

In an embodiment of the present invention, as a result of measuring the content of collagen in injectable compositions solubilized under various temperature (37°C and 55°C) and time (6 hours and 24 hours) conditions, the collagen content obtained at a solubilization temperature of 55°C (87.3 µg/mg under the conditions of 55°C and 6 hours, and 61.8 µg/mg under the conditions of 55°C and 24 hours) was lower than that obtained at a solubilization temperature of 37°C (138.3 µg/mg under the conditions of 37°C and 6 hours and 117.7 µg/mg under the conditions of 37°C and 24 hours) under the solubilization conditions of 6 hours and 24 hours. Therefore, it was confirmed that the collagen content decreased when the solubilization time was 24 hours or more and the solubilization temperature was below 55°C (FIG. 6).

In the present invention, step (b) may be a step of preparing an injectable composition by centrifuging the aqueous solution of step (a) and collecting a solution.

The centrifugation may be performed with about 100 to 500 g for 1 minute to 10 minutes, specifically, about 300 g for 5 minutes, but is not limited thereto.

The prepared injectable composition of the present invention may be used interchangeably with CAM solution or CAM-solution.

The method of the present invention may further include freezing the prepared injectable composition of the present invention after step (b). Storage and handling may be facilitated by the freezing step.

Any freezing method well known in the art may be used therefor without limitation.

The method of the present invention may further include sterilizing the prepared injectable composition of the present invention after step (b).

The sterilization method is as described above, and sterilization may be performed via E-beam irradiation at a dose of about 15 to 25 kGy, but is not limited thereto. By the sterilization method, only impurities may be removed without causing loss or destruction of collagen that is an active ingredient in the injectable composition of the present invention.

In an embodiment of the present invention, when the injectable composition of the present invention is prepared under the solubilization temperature conditions oof 33°C and 35°C, it was confirmed that the solution was injectable both before and after sterilization performed using E-beam at a dose of 15 to 25 kGy (VDmax15).

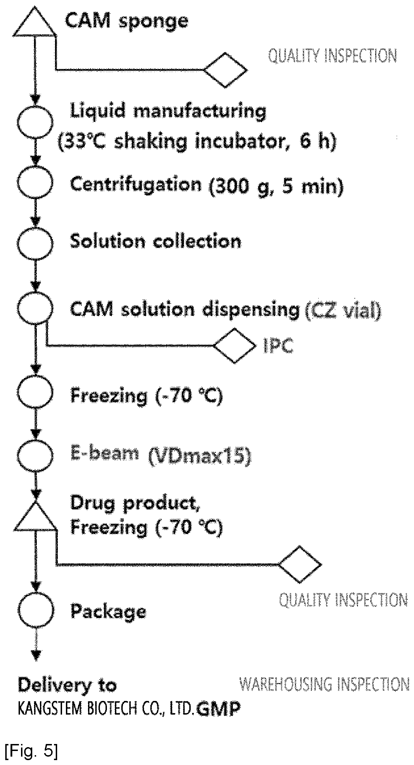

In another embodiment of the present invention, as a result of identifying proteins contained in the injectable composition of the present invention before and after sterilization via SDS-page, it was confirmed that the unsterilized injectable composition mainly contains collagen type II alpha protein corresponding to about 130 kDa, and there was no change in the main protein in spite of a slight decrease after the E-beam sterilization process of VDmax15 (FIG. 5).

Another aspect of the present invention provides an animal cartilage-derived injectable composition for preventing or treating osteoarthritis prepared by the method of the present invention.

The terms used hereinafter are as described above.

The injectable composition of the present invention may include collagen with a concentration of about 120 µg/mg or more based on a total weight of the composition, but is not limited thereto.

The injectable composition of the present invention may induce differentiation of stem cells into chondrocytes.

The injectable composition of the present invention may have effects on repairing tissue and regenerating cartilage tissue.

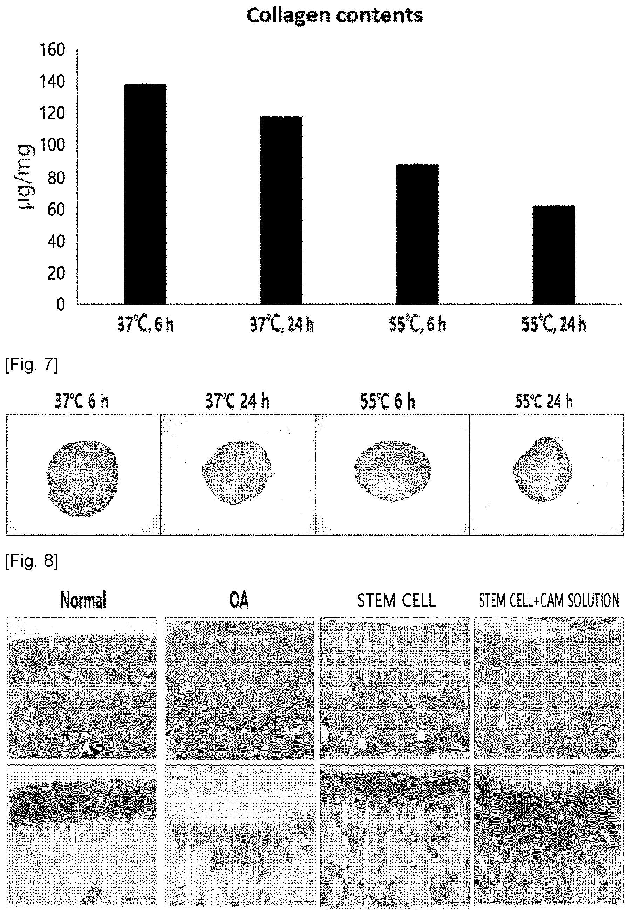

In an embodiment of the present invention, as a result of adding the injectable composition solubilized under various temperature conditions (37°C and 55°C) and time conditions (6 hours and 24 hours) to an umbilical cord blood mesenchymal stem cell-differentiation medium and inducing differentiation into chondrocytes for 2 weeks, it was confirmed that differentiation of the stem cells into chondrocytes was induced in the case of adding the injectable composition solubilized at 37°C for 6 hours to the differentiation medium, but differentiation of the stem cells into chondrocytes was not induced in the case of adding the injectable composition solubilized under the conditions of 37°C and 24 hours, 55°C and 6 hours, and 55°C and 24 hours, respectively, to the differentiation medium (FIG. 7).

The injectable composition of the present invention may be administered together with stem cells.

The phrase "administered together" means that the pharmaceutical composition of the present invention and a stem cell are administered simultaneously or with a time difference either in a dependent or independent manner for prevention or treatment of a disease or alleviation or amelioration of symptoms. The administration together may be used interchangeably with coadministration.

As used herein, the term "stem cells" refers to cells having the ability to differentiate into various tissues, i.e., undifferentiated cells. The stem cells may be pluripotent stem cells, adult stem cells, induced pluripotent stem cells, or embryonic stem cells.

The stem cells may be derived from humans or animals and may be derived from umbilical cord, umbilical cord blood, cartilage, bone marrow, fat, muscle, nerve, skin, amnion, or placenta, but is not limited thereto.

The stem cells of the present invention may be, specifically, adult stem cells. The adult stem cells are undifferentiated cells, which will differentiate into cells of specific tissue if required, and may be extracted from tissue of the adult body such as bone marrow or the brain. The adult stem cells may include at least one selected from the group consisting of mesenchymal stem cells and multipotent stem cells, without being limited thereto.

The stem cells of the present invention may be, more specifically, mesenchymal stem cells, even more specifically, mesenchymal stem cells derived from umbilical cord blood, but are not limited thereto.

The stem cells may be co-administered at a dose of about 1.0×10⁵ cells to 1.0×10⁸ cells, specifically, about 2.5×10⁵ cells to 1.0×10⁸ cells, about 1.0×10⁷ cells to 1.0×10⁸ cells, or about 1.0×10⁷ cells to 5.0×10⁷ cells, more specifically, about 2.5×10⁷ cells, without being limited thereto.

In the case where the injectable composition of the present invention is administered together with the stem cells as described above, the amount of the injectable composition may be from about 1 to about 5 wt%, specifically, from about 1.5 to about 3 wt%, more specifically, about 2 wt%, based on a total weight of a mixed composition administered thereto, but is not limited thereto.

In an embodiment of the present invention, as a result of identifying the ability to regenerate cartilage tissue when umbilical cord blood mesenchymal stem cells (hUCB-MSCs) were administered to joint cavity of an osteoarthritis model of rats induced by causing damage to the anterior cruciate ligament at a dose of 2.5×10⁵ cells/group for 8 weeks (stem cell-administered group) and when the hUCB-MSCs (2.5×10⁵ cells/group) and the CAM solution (2 wt%, 1 mg/group) that is the injectable composition of the present invention were co-administered to the model for 8 weeks (stem cell+CAM solution-administered group), it was confirmed that the stem cell+CAM solution-administered group administered with both the stem cells and the injectable composition of the present invention had a superior ability to regenerate cartilage tissue to that of the stem cell-administered group administered with the hUCB-MSCs alone (FIG. 8).

The injectable composition of the present invention may be a pharmaceutical composition.

The pharmaceutical composition of the present invention has a use for "prevention" and/or "treatment" of osteoarthritis. In terms of the use for prevention, the pharmaceutical composition of the present invention may be administered to an individual having the above-described disease or symptom or an individual suspected to have the risk of outbreak thereof. In terms of the use for treatment, the pharmaceutical composition of the present invention is administered to an individual such as a patient suffering from the disease described herein in an amount sufficient to treat or at least partially stop the disease of symptom described in the present invention. The effective amount for these uses may be determined based on severity of disease or symptoms, progression of disease, previous treatment history, patient's health status, drug sensitivity, and medical judgment of doctors or veterinarians.

The pharmaceutical composition of the present invention may further include an appropriate carrier, excipient, or diluent commonly used in the art. In this regard, although the content of the injectable composition of the present invention contained in the pharmaceutical composition as an active ingredient is not particularly limited, the content of the injectable composition may be from about 1 to 5 wt%, specifically, from about 1.5 to 3 wt%, based on a total weight of the pharmaceutical composition.

The pharmaceutical composition may be formulated in any form selected from the group consisting of sterile aqueous solutions, non-aqueous solvents, suspensions, tablets, pills, powders, granules, capsules, solutions for internal use, emulsions, syrups, lyophilizates, and suppositories, and may also be prepared in various parenteral or oral formulations. For formulations, a diluent or excipient commonly used in the art such as a filler, an extender, a binder, a humectant, a disintegrant, and a surfactant may be used. Solid formulations for oral administration may include tablets, pills, powders, granules, capsules, and the like. The solid formulations may be prepared by mixing the at least one compound with at least one excipient such as starch, calcium carbonate, sucrose, lactose, or gelatin. In addition, a lubricant such as magnesium stearate and talc may also be used in addition to a simple excipient. Liquid formulation for oral administration may be suspensions, formulations for internal use, emulsions, syrups, or the like and may include various excipients such as a humectant, a sweetener, a flavoring agent, and a preservative in addition to a simple common diluent such as water and liquid paraffin. Formulations for parenteral administration may include sterile aqueous solutions, non-aqueous solvents, suspensions, emulsions, lyophilizates, suppositories, and the like. The non-aqueous solvents and suspensions may be propylene glycol, polyethylene glycol, vegetable oil such as olive oil, injectable ester such as ethyl oleate, or the like. Bases for the suppositories may include Witepsol, Macrogol, Tween 61, cacao butter, laurin butter, glycerogelatin, and the like.

In the present invention, the pharmaceutical composition of the present invention may be formulated in a sterile aqueous solution, a non-aqueous solvent, and a suspension, specifically, an injection, for parenteral administration.

The injectable composition of the present invention may be administered to an individual in a pharmaceutically effective amount.

As used herein, the term "pharmaceutically effective amount" refers to an amount sufficient to treat a disease at a reasonable benefit/risk ratio applicable to medical treatment without causing side effects, and the level of the effective amount may be determined according to type of individual, severity of disease, age and gender of individual, type of disease, activity of drug, sensitivity to drug, administration time, administration route, excretion rate, treatment period, factors comprising drugs used in combination or concurrently, and other factors well known in the medical field.

The injectable composition of the present invention may be administered as an individual therapeutic agent or administered in combination with other therapeutic agents, may be administered sequentially or simultaneously with a conventional therapeutic agent, and may be administered in a single dose or multiple doses. It is important to administer a minimum amount capable of obtaining the maximum effect without causing side effects in consideration of all of the above-described factors, which may be easily determined by those skilled in the art. An appropriate dose of the injectable composition of the present invention may vary according to the status and body weight of a patient, severity of disease, formulation of drug, administration route, and administration period and the injectable composition may be administered once a day or many times a day at divided doses.

In the present invention, the injectable composition of the present invention may be administered together with stem cells as described above.

The injectable composition of the present invention may be administered to any individual without limitation for the purpose of preventing or treating osteoarthritis. Any administration methods commonly used in the art may be used without limitation. The injectable composition may be administered to joint cavity (e.g., cartilage), via subcutaneous, intraperitoneal, intrapulmonary, and intranasal routes. For topical treatment, the injectable composition may be administered by a suitable method including intralesional administration, i.e., administration to joint cavity (e.g., cartilage). For example, it may be administered (injected) to joint cavity by injection, without being limited thereto.

The injectable composition of the present invention may generally be administered at a concentration of about 1 to 5 wt%, specifically, a concentration of about 1.5 to 3 wt%, without being limited thereto.

Still another aspect of the present invention provides a method for preventing or treating osteoarthritis, the method including administering an injectable composition of the present invention to joint cavity of an individual.

The terms used herein are as described above.

As used herein, the term "individual" refers to any animal having osteoarthritis or at the risk of developing osteoarthritis. By administering the injectable composition of the present invention to an individual suspected to have osteoarthritis, the individual may be effectively treated. The individual is not particularly limited and may be animals such as monkeys, dogs, cats, rabbits, guinea pigs, rats, mice, cows, sheep, pigs, goats, and birds, without being limited thereto.

As used herein, the term "administration" refers to introduction of the injectable composition of the present invention into an individual suspected to have osteoarthritis by any suitable method, and the administration route may be various parenteral routes as long as the injectable composition arrives target tissue. The injectable composition of the present invention may be administered in a pharmaceutically effective amount and the pharmaceutically effective amount is as described above.

Additionally, in the present invention, the injectable composition of the present invention may be administered together with stem cells as described above.

### [Mode for Invention]

Hereinafter, the present invention will be described in more detail with reference to the following examples. However, the following examples are merely presented to exemplify the present invention, and the scope of the present invention is not limited thereto. These examples are provided to fully convey the concept of the invention to those skilled in the art.

### Example 1. Identification of Injectable Raw Material and Solubilization Condition

In order to obtain a process of manufacturing a CAM solution in an injectable form, three raw materials (sterilized CAM-WS powder, unsterilized CAM-WS sponge, and sterilized CAM-WS sponge) (produced by ATEMs, Korea) were eluted into a shaking incubator under three solubilization temperature conditions (4°C, 25°C, and 37°C) and three solubilization time conditions (3 hours, 6 hours, and 18 hours) and centrifuged with 300 g for 5 minutes to prepare CAM solutions, and then injectability of each solution was analyzed. In the above-described experiment, sterilization was performed with gamma-rays at a dose of 25 to 40 kGy according to the VDmax25 method.

As a result, as shown in FIG. 1 and FIG. 2 showing CAM solutions prepared under three solubilization temperature conditions (4°C, 25°C, and 37°C) for 6 hours, it was confirmed that injectable CAM solutions could not be prepared by using the unsterilized CAM-WS sponge as a raw material, and injectable CAM solutions were prepared under the solubilization temperature condition of 37°C by using the sterilized CAM-WS powder and the sterilized CAM-WS sponge as raw materials.

### Example 2. Identification of Injectable Sterilization Condition

In order to obtain a process of manufacturing a CAM solution in an injectable form even after sterilization, CAM solutions were prepared using the sterilized CAM-WS sponge under three solubilization temperature conditions (30°C, 33°C, and 35°C) and two solubilization time conditions (6 hour-elution and 18-hour elution) and sterilized by E-beam irradiation at a dose of 15 to 25 kGy according to the VDmax15 method, and then injectability of each solution was analyzed.

As a result, as shown in FIG. 3, it was confirmed that the CAM solution prepared at 30°C was not injectable both before and after sterilization, and the CAM solutions prepared at 33°C and 35°C were injectable both before and after sterilization.

Based thereon, it was confirmed that the CAM solution, prepared by a method including solubilization at a temperature of 30°C or higher, had stability against sterilization.

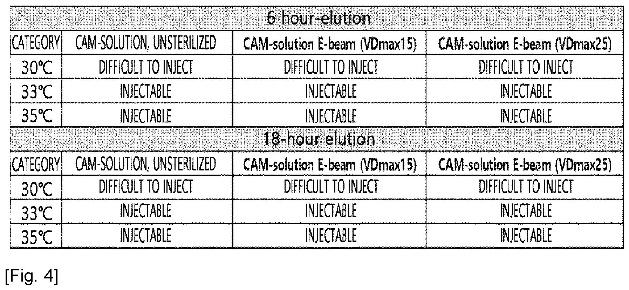

Based on the results of Example 2, the process of manufacturing a CAM solution in an injectable form even after sterilization was derived and a representative example of the manufacturing process is shown in FIG. 4.

### Example 3. Identification of Collagen Content Before and After Sterilization in Injection-manufacturing Process

Proteins contained in the CAM solution prepared in a process shown in FIG. 4 were identified before and after sterilization by SDS-page.

As a result, as shown in FIG. 5, it was confirmed that the unsterilized CAM solution mainly contains collagen type II alpha protein corresponding to about 130 kDa, and there was no change in the mainly contained protein in spite of a slight decrease after the E-beam sterilization process of VDmax15.

Based thereon, in the process of manufacturing the CAM solution as an injectable formulation, an optical temperature of higher than 30°C and lower than 55°C and an optimal time of less than 24 hours were selected for solubilization.

### Example 4. Identification of Collagen Content According to Solubilization Process Condition

In order to identify differences in collagen contents in accordance with solubilization process conditions, the sterilized CAM-WS sponge was eluted into a shaking incubator under two solubilization temperature conditions (37°C and 55°C) and two solubilization time conditions (6 hours and 24 hours) and centrifuged with 300 g for 5 minutes to prepare CAM solutions, and then the collagen content of each solution was identified.

As a result, as shown in FIG. 6, it was confirmed that the collagen content obtained at 55°C (87.3 µg/mg under the conditions of 55°C and 6 hours, and 61.8 µg/mg under the conditions of 55°C and 24 hours) was lower than that obtained at 37°C (138.3 µg/mg under the conditions of 37°C and 6 hours and 117.7 µg/mg under the conditions of 37°C and 24 hours) under the solubilization conditions of 6 hours and 24 hours. Therefore, it was confirmed that the collagen content decreased when the solubilization time was 24 hours or more and the solubilization temperature was 55°C or higher.

### Example 5. Comparison of Ability to Differentiate into Chondrocytes According to Solubilization Process Condition

After adding the CAM solution prepared under the same solubilization conditions as those of Example 4 to a differentiation medium of umbilical cord blood mesenchymal stem cells, cartilage differentiation was induced for 2 weeks, and the cells were observed by Safranin-O staining.

As a result, as shown in FIG. 7, differentiation of stem cells into chondrocytes was induced when the CAM solution prepared by the solubilization process conditions at 37°C for 6 hours was added to the differentiation medium. On the contrary, differentiation of stem cells into chondrocytes was not induced when the CAM solutions solubilized under the conditions of 37°C and 24 hours, 55°C and 6 hours, and 55°C and 24 hours were added to the differentiation medium.

As can be seen from the above results, it was confirmed that the injectable composition prepared by the process derived in the above-described examples had the ability to differentiate into chondrocytes so as to be appropriate as a therapeutic agent for osteoarthritis.

### Example 6. Effect on Enhancing Ability to Regenerate Cartilage

In order to identify effects of the CAM solutions prepared under the same solubilization conditions as those of Example 4 on enhancing the ability to regenerate cartilage, an osteoarthritis model of rats induced by causing damage to the anterior cruciate ligament was prepared.

Specifically, 65 rats determined to have no abnormalities in medical inspection during an acclimatization period were anesthetized by intraperitoneally administering a mixture of ketamine at 20 mg/kg (Yuhan Corp., Seoul, Korea) and xylazine at 3 mg/kg (Rompun; Bayer Korea Corp., Seoul, Korea). Hair on right articulatio genu and tibial metaphysis of each rat was removed using a grainer and disinfected using betadine, and then a longitudinal incision of about 1 cm was made at a tibial tubercle region (medial parapatellar approach) in articulatio genu. The articular capsule under the incised region was exposed and incised, and the articulatio genu was located outward, and then the knee was bent to expose the anterior cruciate ligament and meniscus. The medial parenchyma of the anterior cruciate ligament was completely cut using micro-scissors and loosened support of the tibia by ligaments was confirmed using a positive anterior drawer's test. Then, the articular capsule and subcutaneous tissue were continuously sutured with absorbable sutures 4-0 Monosyn^{®} (B. Braun Surgical SA, Rubi, Spain), and the skin was simply sutured with non-absorbable sutures 4-0 Nylon (Blue nylon, Ailee Co., Ltd., South Korea). After the surgery, the surgical site was disinfected with betadine for 3 to 4 days to prevent infection and intramuscularly injected with Cefazolin (Cefazol^{®}, Eaglevet Tech Co., Ltd., Korea) once a day at a dose of 100 mg/kg for three days. All experimental animals were allowed to move freely within a breeding cage fir 8 weeks without separate bandage treatment on the surgical site.

The ability to regenerate cartilage tissue was evaluated in a group in which umbilical cord blood mesenchymal stem cells (hUCB-MSCs) were administered to joint cavity of the osteoarthritis model of rats induced by causing damage to the anterior cruciate ligament at a dose of 2.5×10⁵ cells/group for 8 weeks (stem cell-administered group) and in a group in which the hUCB-MSCs (2.5×10⁵ cells/group) and the CAM solution (2 wt%, 1 mg/group) prepared by the process shown in FIG. 4 were co-administered to the model for 8 weeks (stem cell+CAM solution-administered group). At week 8 of administration, cartilage tissue was observed by Safranin-O staining.

As a result, as shown in FIG. 8, it was confirmed that the group administered with the CAM solution together with the hUCB-MSCs had superior ability to regenerate cartilage tissue to the stem cell-administered group treated only with the hUCB-MSCs.

Based on the above-described results of the examples, the injectable composition of the present invention may be used as a therapeutic agent for arthritis by alleviating osteoarthritis not only because the porcine cartilage-derived extracellular matrix and collagen protect articular cartilage tissue but also because an environment capable of regenerating damaged articular tissue is created by inducing differentiation of intra-articular stem cells into chondrocytes.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A method of manufacturing an injectable composition for preventing or treating osteoarthritis, the method comprising:
(a) solubilizing a composition including an animal cartilage-derived acellular extracellular matrix (cartilage acellular matrix, CAM) by stirring the composition at a temperature higher than 30°C and lower than 55°C; and
(b) preparing an injectable composition by centrifuging the aqueous solution prepared in step (a) and collecting a solution.

2. The method according to claim 1, wherein the step (a) is performed for less than 24 hours.

3. The method according to claim 1, wherein the CAM is sterilized.

4. The method according to claim 1, further comprising sterilizing the prepared injectable composition.

5. The method according to claim 1, wherein the animal is a pig.

6. An injectable composition for preventing or treating osteoarthritis prepared according to the method of claim 1.

7. The injectable composition according to claim 6, wherein the injectable composition includes collagen at a concentration of 120 µg/mg or more based on a total weight of the composition.

8. The injectable composition according to claim 6, wherein the injectable composition induces differentiation of stem cells into chondrocytes.

9. The injectable composition according to claim 6, wherein the injectable composition has effects on repairing tissue and regenerating cartilage tissue.

10. The injectable composition according to claim 6, wherein the injectable composition is administered together with stem cells.

11. The injectable composition according to claim 10, wherein the stem cells are administered at a dose of 1.0×10⁵ cells to 1.0×10⁸ cells.

12. A method for preventing or treating osteoarthritis, the method comprising administering the injectable composition of claim 6 to a joint cavity of an individual.

13. The method according to claim 12, wherein the injectable composition is administered together with stem cells.

14. The method according to claim 13, wherein the stem cells are administered at a dose of 1.0×10⁵ cells to 1.0×10⁸ cells.
